# EUROPEAN PATENT APPLICATION

(11) **EP 1 985 231 A1**
(43) Date of publication of application: **29.10.2008**
(21) Application number: 06834467.0
(22) Date of filing: 12.12.2006
(51) Int. Cl.: A61B 5/0404

(54) **PORTABLE ELECTROCARDIOGRAPH**

(30) Priority: 10.02.2006 JP 2006034162
(71) Applicant: Omron Healthcare Co., Ltd., Kyoto 615-0084 (JP)
(72) Inventor: SAKODA, Yusaku Omron Healthcare Co., Ltd., Kyoto-shi Kyoto 615-0084 (JP); YAMAMOTO, Norihito c/o Omron Healthcare Co., Ltd., Kyoto-shi Kyoto 615-0084 (JP)
(74) Representative: Wilhelms · Kilian & Partner Patentanwälte
(86) International application number: PCT/JP2006/324712
(87) International publication number: WO 2007/091372

(57) **Abstract**

In a portable electrocardiograph (100), including a negative electrode (121) to be contacted by a hand of a subject and a positive electrode (122) to be contacted to a predetermined measurement site of a subject, for measuring an electric signal detected by the negative electrode (121) and the positive electrode (122) as an electrocardiographic waveform, springs (190a, 190b, 190c, 190d) for supporting the positive electrode (122) in a freely oscillating manner are arranged in a body unit (110) including the negative electrode (121) and a processing circuit for measurement. Thus, a portable electrocardiograph that excels in handleability and that enhances the contacting stability between the electrode and the measurement site can be achieved.

## Description

### TECHNICAL FIELD

The present invention relates to electrocardiographs, in particular, to a portable electrocardiograph that can be carried around and that can easily measure an electrocardiographic waveform.

### BACKGROUND ART

The electrocardiogram of a patient is generally used to diagnose arrhythmia, and ischemic heart disease such as cardiac angina and myocardial infarction. Various configurations have been proposed for the electrocardiograph used to obtain the electrocardiogram. One of which is a portable electrocardiograph that can be carried around, and that measures and stores the electrocardiographic waveform when the subject himself/herself contacts the electrode to the body when a certain symptom to be measured occurs. This portable electrocardiograph is classified as an event-type electrocardiograph, and is distinguished from a halter-type electrocardiograph in which the electrode is attached to the body of the patient beforehand, and the electrocardiographic waveform is successively measured and recorded while going about his/her daily life.

In such portable electrocardiograph, proposal has been conventionally made to accurately measure the electrocardiographic waveform. For instance, Japanese Laid-Open Patent Publication No. 2005-46215 (Patent Document 1) discloses an integrated portable electrocardiograph for measuring the electrocardiographic waveform by having the subject grip the body of the electrocardiograph, and press the measurement electrode (positive electrode) against a predetermined measurement site (chest region). In the portable electrocardiograph disclosed in Patent Document 1, a non-electrode region formed to a flat shape is arranged so as to surround an electrode region at where the positive electrode is positioned to enhance the contact stability between the contacting face of the positive electrode and the measurement site. Thus, even if the hand gripping the electrocardiograph or the measurement site slightly moves, the fluctuation of the contact site will occur in the non-electrode region and will hardly occur in the electrode region.

In addition, Japanese Laid-Open Patent Publication No. 2005-185756 (Patent Document 2) describes carrying out the measurement with the measurement electrode pulled out by a connection cable from a body unit attached to the measurement site. The measurement electrode is then fixed to the measurement site without having the subject press the body unit against the measurement site, and thus the contact stability between the electrode and the measurement site can be maintained.
[Patent Document 1] Japanese Laid-Open Patent Publication No. 2005-46215
[Patent Document 2] Japanese Laid-Open Patent Publication No. 2005-185756

### DISCLOSURE OF THE INVENTION

### PROBLEMS TO BE SOLVED BY THE INVENTION

However, in the integrated portable electrocardiograph having the configuration described in Patent Document 1, the direction to which the subject presses the body of the electrocardiograph against the measurement site needs to coincide with the normal direction at the surface of the measurement site to a certain extent. Thus, if the hand or the arm gripping the electrocardiograph lowers, and the direction of pressing the electrocardiograph against the measurement site greatly shifts from the normal direction at the surface of the measurement site during the measurement, it is sometimes insufficient with only the friction of a projection to satisfactorily maintain the contact stability between the contacting face of the positive electrode and the measurement site.

In addition, in Patent Document 2, the measurement can be made by inserting the connection cable to a jack of the body unit. Thus, even if a certain symptom occurs and measurement desirably needs to be made right away, the connection cable needs to be inserted to the jack, and thus the subject might feel inconvenience in handling.

Furthermore, in the integrated portable electrocardiograph disclosed in Patent Document 1, it is sometimes difficult to press the measurement electrode so as to be parallel to the surface of the measurement site depending on the build (corpulent, slender) of the subject and the flexibility of the body.

In view of solving the above problems, it is an object of the present invention to provide a portable electrocardiograph that excels in handleability and that enhances the contact stability between the electrode and the measurement site.

It is another object of the present invention to provide a portable electrocardiograph that excels in handleability, and that satisfactorily contacts the electrode and the measurement site irrespective of the direction of approaching the electrocardiograph body to the measurement site.

### MEANS FOR SOLVING THE PROBLEMS

A portable electrocardiograph based on the present invention includes a first electrode to be contacted by a hand of a subject; a second electrode to be contacted to a predetermined measurement site of the subject; a measurement unit for measuring an electric signal detected by the first electrode and the second electrode as an electrocardiographic waveform; a first housing including the first electrode and the measurement unit; and an oscillating and supporting mechanism, arranged in the first housing, for supporting the second electrode in a freely oscillating manner.

In the portable electrocardiograph based on the present invention, the first housing preferably has an electrode formed face arranged with the first electrode, and a first face positioned on the opposite side of the electrode formed face; and the second electrode preferably has a contacting face which contacts the measurement site, and a second face positioned on the opposite side of the contacting face. In this case, the oscillating and supporting mechanism preferably couples the first face and the second face.

In the portable electrocardiograph based on the present invention, the portable electrocardiograph preferably further includes a second housing arranged with the second electrode. In this case, the oscillating and supporting mechanism preferably couples the first housing and the second housing. Furthermore the first housing preferably has a first electrode formed face arranged with the first electrode, and a first face positioned on the opposite side of the first electrode formed face; and the second housing preferably has a second electrode formed face arranged with the second electrode, and a second face positioned on the opposite side of the second electrode formed face. The oscillating and supporting mechanism preferably couples the first face and the second face.

In the portable electrocardiograph based on the present invention, the portable electrocardiograph preferably further includes a detection unit for detecting whether or not the second electrode is pressed against the measurement site by a subject. In this case, the detection unit preferably includes a button arranged on the first face and projected out towards the second face side in an unused state, and a push detection unit for detecting whether or not the button is pushed; and the push detection unit preferably instructs start of measurement by the measurement unit when the pushing of the button is detected.

In the portable electrocardiograph based on the present invention, the oscillating and supporting mechanism preferably includes one of an elastic body, a fluid bag containing fluid, or a universal joint.

### EFFECTS OF THE INVENTION

According to the present invention, a portable electrocardiograph that excels in handleability, and that enhances the contacting stability between the electrode and the measurement site is achieved.

Furthermore, according to the present invention, a portable electrocardiograph that excels in handleability and that can satisfactorily contact the electrode and the measurement site irrespective of the direction of approaching the electrocardiograph body to the measurement site is achieved.

### BRIEF DESCRIPTION OF THE DRAWINGS

Fig. 1 is a schematic perspective view of a portable electrocardiograph according to a first embodiment of the present invention.
Fig. 2 is a front view of the portable electrocardiograph according to the first embodiment of the present invention.
Fig. 3 is a top view of the portable electrocardiograph according to the first embodiment of the present invention.
Fig. 4 is a bottom view of the portable electrocardiograph according to the first embodiment of the present invention.
Fig. 5 is a right side view of the portable electrocardiograph according to the first embodiment of the present invention.
Fig. 6 is a left side view of the portable electrocardiograph according to the first embodiment of the present invention.
Fig. 7 is a perspective view showing a measuring position to be taken by a subject when measuring an electrocardiographic waveform using the portable electrocardiograph according to the first embodiment of the present invention.
Fig. 8 is a view seen from above of the measuring position to be taken by the subject when measuring the electrocardiographic waveform using the portable electrocardiograph according to the first embodiment of the present invention.
Fig. 9 is a block diagram showing a hardware configuration of the portable electrocardiograph according to each embodiment of the present invention.
Fig. 10 is a flowchart showing a flow of process executed by a CPU in the portable electrocardiograph according to each embodiment of the present invention.
Fig. 11 is a view showing one example of a screen display of a measurement result in each embodiment of the present invention.
Fig. 12 is a view showing one example of a display screen of a measurement result of an electrocardiographic waveform stored in a memory in each embodiment of the present invention.
Fig. 13 is a view showing a state in which the springs are supporting the electrode unit in a freely oscillating manner in the first embodiment of the present invention.
Fig. 14 is a view showing one example of an outer appearance of a portable electrocardiograph according to a second embodiment of the present invention.
Fig. 15 is a view showing a state where the measurement button is being pushed in the second embodiment of the present invention.
Fig. 16 is a view showing one example of an outer appearance of a portable electrocardiograph according to a variant of the second embodiment of the present invention.
Fig. 17 is a view showing a state in which the measurement button is pushed in the variant of the second embodiment of the present invention.
Fig. 18 is a view showing one part of an outer appearance of a portable electrocardiograph according to a third embodiment of the present invention.
Fig. 19 is a view showing a state in which a universal joint is supporting the electrode unit in a freely oscillating manner in the third embodiment of the present invention.
Fig. 20 is a view showing one part of an outer appearance of a portable electrocardiograph according to a fourth embodiment of the present invention.
Fig. 21 is a view showing a state in which a fluid bag is supporting the electrode unit in a freely oscillating manner in the fourth embodiment of the present invention.
Fig. 22 is a perspective view showing one part of an outer appearance of a portable electrocardiograph according to a fifth embodiment of the present invention.
Fig. 23 is a front view showing one part of an outer appearance of the portable electrocardiograph according to the fifth embodiment of the present invention.
Fig. 24 is a view showing a state in which the springs are supporting the positive electrode in a freely oscillating manner in the fifth embodiment of the present invention.

### DESCRIPTION OF SYMBOLS

- 100, 100A, 100B, 100C, 100D, 100E: Portable electrocardiograph
- 110, 110A, 110B, 110C, 110D, 110E: Body unit
- 111: Front face
- 112: Rear face
- 113: Upper face
- 114: Lower face
- 115: Right side face
- 116: Left side face
- 117: Electrode unit
- 120: Electrode unit
- 121: Negative electrode
- 122: Positive electrode
- 123: Indifferent electrode
- 130: Open/close cover
- 132: External storage medium
- 140: Operation unit
- 141: Power button
- 142, 142A: Measurement button
- 143: Menu button
- 144: Determination button
- 145: Left scroll button
- 146: Right scroll button
- 148: Display unit
- 150: Processing circuit
- 151: Amplifier circuit
- 152: Filter circuit
- 153: A/D converter
- 154: CPU
- 155: Memory
- 160: Power
- 170: Timer
- 180: Flexible substrate
- 181: Lead wire
- 190a, 190b, 190c, 190d, 191: Spring
- 192: Universal joint
- 193: Fluid bag

### BEST MODE FOR CARRYING OUT THE INVENTION

The embodiments of the present invention will now be described in detail with reference to the drawings. Same reference numerals are denoted for the same or corresponding components throughout the drawings, and the description thereof will not be repeated.

### [First embodiment]

### (Regarding outer appearance)

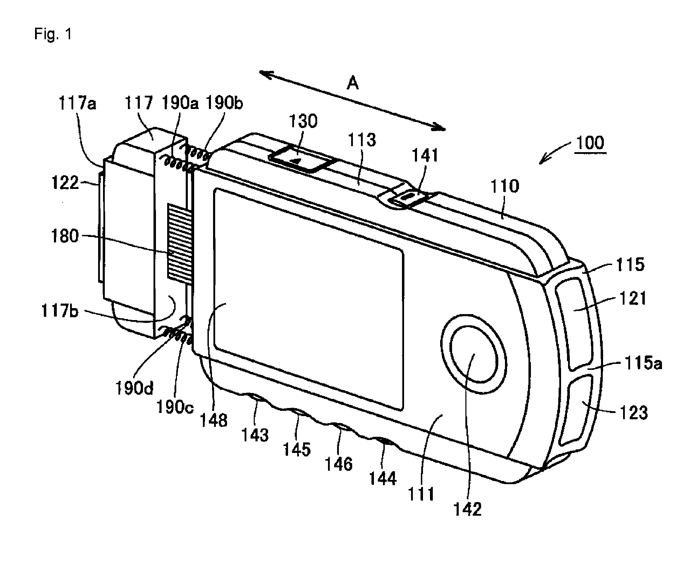

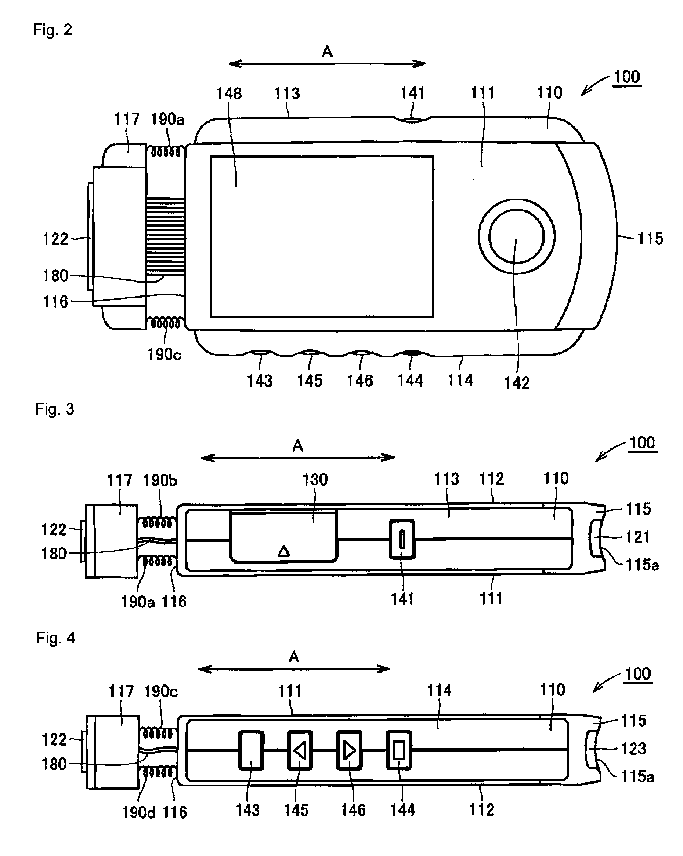

First, the outer appearance structure of a portable electrocardiograph according to the present embodiment will be described. Fig. 1 is a schematic perspective view of a portable electrocardiograph 100 according to the present embodiment. Figs. 2 to 6 are front view, top view, bottom view, right side view, and left side view of the portable electrocardiograph 100 according to the present embodiment.

The portable electrocardiograph 100 according to the present embodiment is miniaturized and made lighter to a size and weight capable of being held by one hand so as to excel in handleability. As shown in Figs. 1 to 6, the portable electrocardiograph 100 includes a body unit 110 having an outer shape of a flat and elongate substantially rectangular solid shape extending in the direction of arrow A in the figure, and an electrode unit 117 arranged with a positive electrode 122 to be hereinafter described.

The portable electrocardiograph 100 includes a supporting member, arranged in the body unit 110, for supporting the positive electrode 122 in a freely oscillating manner. In the portable electrocardiograph 100 according to the present embodiment, a stretchable elastic body such as springs 190a, 190b, 190c, and 190d including coil spring are used for the supporting member. Here, the term "support" includes both concepts of direct support and indirect support. In the present embodiment, the springs 190a, 190b, 190c, and 190d or supporting members directly support the electrode unit 117 in a freely oscillating manner, so that the body unit 110 and the electrode unit 117 are coupled.

As shown in Figs. 1 and 2, a measurement button 142 for instructing the start of measurement is arranged close to one end in a longitudinal direction (direction of arrow A in the figure) of a front face 111 of the body unit 110. A display unit 148 is arranged close to the other end of the front face 111 of the body unit 110. The display unit 148 is configured by a liquid crystal display and the like, and displays measurement result etc.

As shown in Figs. 1 and 3, a power button 141 for instructing ON/OFF of a power 160 is arranged at a predetermined position of an upper face 113 of the body unit 110. An open/close cover 130 or a lid body is arranged at a predetermined position of the upper face 113 of the body unit 110. The open/close cover 130 is arranged to cover an external storage medium slot (not shown) in the closed state, and is attached to the body unit 110 in a freely opening/closing manner.

As shown in Figs. 1 and 4, a menu button 143, a determination button 144, a left scroll button 145, and a right scroll button 146 are arranged at predetermined positions of a lower face 114 of the body unit 110. The menu button 143 is an operation button for instructing display of a menu of the portable electrocardiograph 100, and the determination button 144 is an operation button for executing a setting operation associated with the information displayed on the display unit 148. The left scroll button 145 and the right scroll button 146 are operation buttons for scrolling and displaying graphs of the measurement result, guide information and the like displayed on the display unit 148.

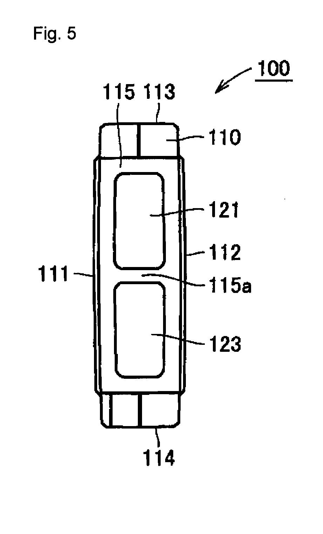

As shown in Figs. 1 and 5, a negative electrode 121, which is one of a pair of measurement electrodes, to be touched by the hand of the subject, and an indifferent electrode 123 for obtaining a potential that becomes the reference of potential change of the body are arranged on a right side face 115 positioned at one end in the longitudinal direction of the body unit 110. The right side face 115 has a smoothly curved shape so that the forefinger of the right hand of the subject fits thereto when the subject takes a measuring position to be hereinafter described. Furthermore, a concave part 115a extending in the up and down direction is formed in the right side face 115. The concave part 115a has a shape for receiving the forefinger of the right hand of the subject.

The negative electrode 121 and the indifferent electrode 123 described above are formed by a conductive member. The negative electrode 121 and the indifferent electrode 123 are arranged so that the surfaces thereof are exposed on the outer surface of the body unit 110 in the concave part 115a formed in the right side face 115. The negative electrode 121 is positioned closer to the upper face 113 of the right side face 115, and the indifferent electrode 123 is positioned closer to the lower face 114 of the right side face 115.

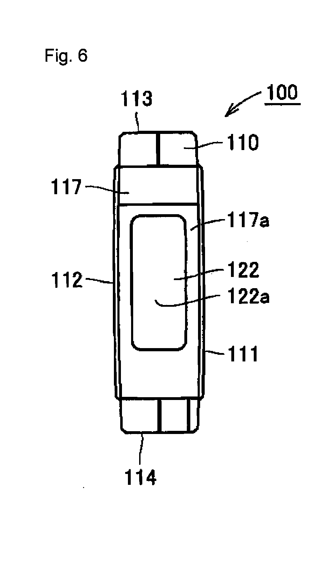

As shown in Figs. 1 and 6, the positive electrode 122, which is the other electrode of the pair of measurement electrodes, to be contacted to a predetermined site of the subject is arranged on a face 117a at one end of the electrode unit 117. In the present embodiment, the face 117a arranged with the positive electrode 122 of the electrode unit 117 is referred to as "electrode formed face 117a".

As shown in Figs. 1 to 4, the face (i.e., face 117b at the other end of the electrode unit 117) positioned on the side opposite to the electrode formed face 117a of the electrode unit 117, and the face (i.e., left side face 116 of the body unit 110) positioned on the side opposite to the face (right side face 115) arranged with the negative electrode 121 and the indifferent electrode 123 are coupled by the springs 190a, 190b, 190c, and 190d. More specifically, the face 117b of the electrode unit 117 and the left side face 116 of the body unit 110 respectively have a substantially rectangular shape, and the springs 190a, 190b, 190c, and 190d couples the respective four corners of the faces 117b, 116. The springs 190a, 190b, 190c, and 190d couple the faces such that the face 117b of the electrode unit 117 and the left side face 116 of the body unit 110 become substantially parallel in a unused state (state in which force is not externally applied). Thus, the springs 190a, 190b, 190c, and 190d indirectly support the positive electrode 122 in a freely oscillating manner.

As shown in Figs. 1 to 4, a flexible substrate 180 is arranged between the body unit 110 and the electrode unit 117. The flexible substrate 180 is a member for electrically connecting a processing circuit 150 (to be hereinafter described) arranged inside the body unit 110 and the positive electrode 122 arranged in the electrode unit 117.

In the portable electrocardiograph 100 of the present embodiment, the positive electrode 122 and the processing circuit 150 are electrically connected by the flexible substrate 180, but it is not limited to such mode. For instance, a connection cable such as lead wire for electrically connecting the positive electrode 122 and the processing circuit 150 may be arranged to pass through the interior portion of the springs 190a, 190b, 190c, and 190d in the axial direction.

### (Regarding measuring position)

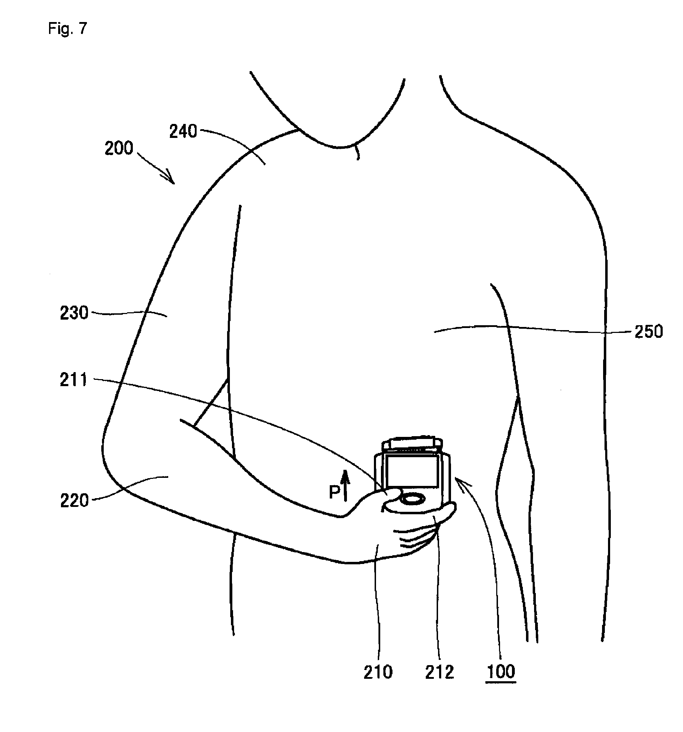

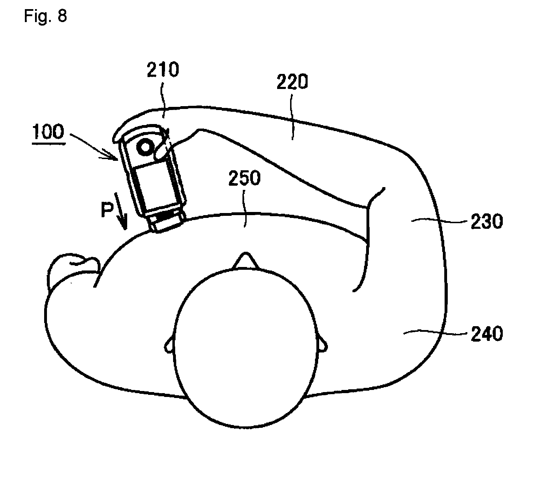

The measuring position of when the subject measures the electrocardiographic waveform using the portable electrocardiograph 100 of the present embodiment will now be described. Fig. 7 is a perspective view showing the measuring position of the subject, and Fig. 8 is a view of such measuring position seen from above.

As shown in Figs. 7 and 8, the subject 200 directly contacts the positive electrode 122 of the electrode unit 117 to the skin on the fifth intercostal space in the anterior axillary line positioned at the lower part of the left side of the chest region 250 while gripping the right side face 115 side of the body unit 110 of the portable electrocardiograph 100 with the right hand 210. The direction (direction of arrow P in the figure) the subject 200 approaches the body unit 110 (left side face 116 thereof) to the chest region 250 to press the positive electrode 122 against the chest region 250 is hereinafter referred to as "moving direction". The direction the positive electrode 122 is pressed against the chest region 250 is referred to as "contacting direction". The moving direction corresponds to the normal direction of the left side face 116 of the body unit 110, and the contacting direction corresponds to the normal direction of the contacting face 122a of the positive electrode 122.

The subject 200 pushes the measurement button 142 arranged on the front face 111 of the body unit 110 with the thumb 211 of the right hand 210 with the positive electrode 122 contacting the surface of the chest region 250. The electrocardiographic waveform is measured while maintaining the relevant state for about a few dozen seconds.

A state in which the negative electrode 121 and the indifferent electrode 123 positioned on the right side face 115 of the body unit 110 of the electrocardiograph 100 contact the forefinger 212 of the right hand 210 of the subject 200 and the positive electrode 122 arranged on the electrode unit 117 contacts the chest region 250 of the subject 200 is obtained by taking the above measuring position. Thus, a measurement circuit is configured in the body of the subject in the order of the right hand 210 contacting the negative electrode 121, the forearm 220 non-contacting the chest region 250, the upper arm 230 and the right shoulder 240 non-contacting the chest region 250, and the chest region 250 contacting the positive electrode 122.

### (Regarding configuration)

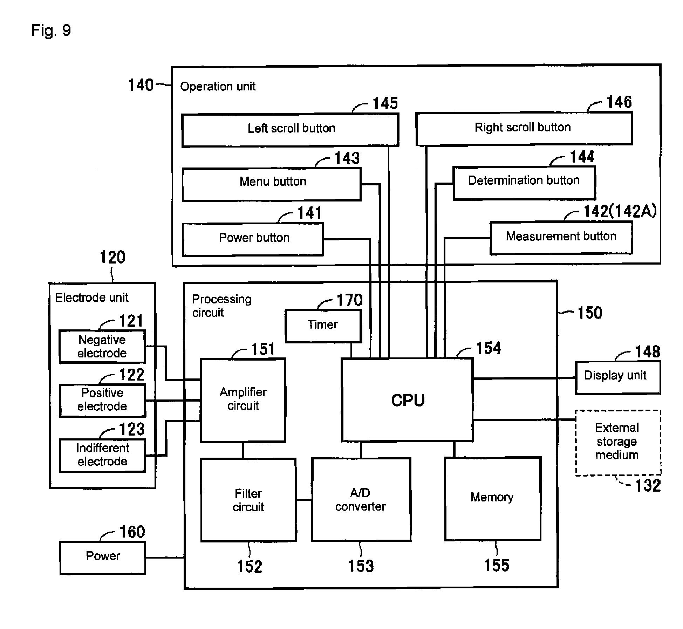

The hardware configuration of the portable electrocardiograph 100 according to the present embodiment will now be described. Fig. 9 is a block diagram of the portable electrocardiograph 100 according to the present embodiment.

With reference to Fig. 9, the portable electrocardiograph 100 according to the present embodiment mainly includes the electrode unit 120, the operation unit 140, and the processing circuit 150. The electrode unit 120 is configured by the negative electrode 121, the positive electrode 122, and the indifferent electrode 123 described above. In the portable electrocardiograph 100 according to the present embodiment, the operation unit 140 includes the power button 141, the measurement button 142, the menu button 143, the determination button 144, the left scroll button 145, and the right scroll button 146.

The processing circuit 150 incorporates a circuit for processing a living body electric signal detected by the electrode unit 120 to measure the same as an electrocardiographic waveform, and includes an amplifier circuit 151 for amplifying the living body electric signal detected by the electrode unit 120, a filter circuit 152 for removing noise component from the living body electric signal detected by the electrode unit 120, an A/D (Analog/Digital) converter 153 for converting an analog signal to a digital signal, a CPU (Central Processing Unit) 154 for performing various calculations, a memory 155 including a ROM (Read Only Memory) and a RAM (Random Access Memory) for storing electrocardiographic information, and a timer 170 for outputting time data timed through a timing operation to the CPU 154. In this case, the amplifier circuit 151 differentially amplifies an output voltage signal (living body electric signal) of the negative electrode 121 and the positive electrode 122 based on the output voltage signal of the indifferent electrode 123, and outputs the same. In addition, the filter circuit 152, for example, uses a band pass filter having a pass band of between 0.5 Hz and 35 Hz.

The processing circuit 150 is connected with the electrode unit 120 and the operation unit 140, and in addition, connected with the display unit 148 and the power 160. When an external storage medium is inserted to the slot for inserting the external storage medium, the external storage medium 132 is also connected to the processing circuit 150.

The CPU 154 executes an analysis process of the digital signal input from the A/D converter 153. The CPU 154 receives command signals from various operation buttons in the operation unit 140, and executes a process corresponding to the received command signal. The CPU 154 executes write and readout of information to and from the memory 155. The CPU 154 performs display control on the display unit 148.

### (Regarding operation)

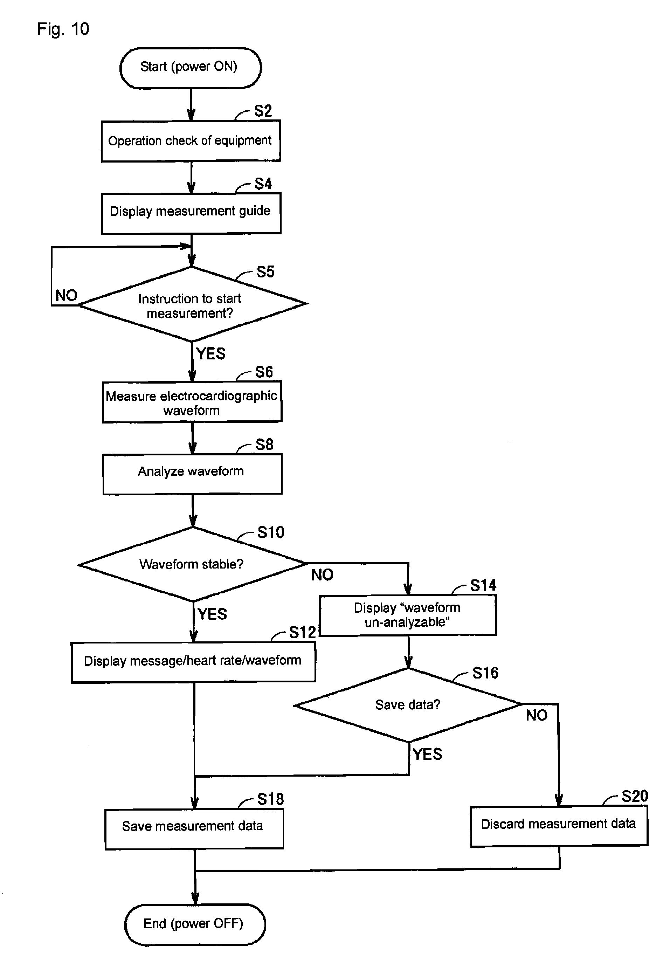

Fig. 10 is a flowchart showing a flow of processes executed by the CPU in the portable electrocardiograph 100 of the present embodiment. The processes shown in the flowchart of Fig. 10 are stored in advance in the ROM of the memory 155 as a program, and are realized by causing the CPU 154 to read out and execute the program.

With reference to Fig. 10, when the power button 141 is pushed by the subject and the power is turned ON, the operation check of the equipment is performed (step S2). The CPU 154 then displays a measurement guide on the display unit 148 (step S4). For instance, information such as the position the subject or the user needs to take for measurement are displayed as the measurement guide.

Subsequently, the CPU 154 determines whether or not instruction to start the measurement is externally made (step S5). Specifically, whether or not the measurement button 142 is pushed by the subject is determined. The CPU 154 waits until the instruction to start the measurement is made (NO in step S5).

If determined that the instruction to start the measurement is made (YES in step S5), the CPU 154 starts the measurement process of the electrocardiographic waveform, and analyzes the measured electrocardiographic waveform (steps S6, S8). The electrocardiographic waveform converted to a digital signal by the A/D converter 153 is temporarily recorded in the RAM of the memory 155. The analysis of the electrocardiographic waveform is a process of detecting presence of shape feature showing such as arrhythmia, ischemia of cardiac muscle, presence of periodic feature showing such as slow pulse and fast pulse, presence of an un-analyzable waveform due to such as noise and baseline fluctuation, and the like from the electrocardiographic waveform converted to a digital signal, and analyzing the detection result. The measurement and the analysis of the electrocardiographic waveform may be carried out through known procedures.

As a result of waveform analysis, the CPU 154 determines whether or not the measured electrocardiographic waveform is stable, that is, the presence of an un-analyzable waveform (step S10). If determined that the electrocardiographic waveform is stable (YES in step S10), the process proceeds to step S12. On the other hand, if determined that the electrocardiographic waveform is not stable (NO in step S10), the process proceeds to step S14.

In step S12, the CPU 154 edits the analysis result of the electrocardiographic waveform to a message, and displays the message on the display unit 148. In this case, the heart rate per unit time is displayed with the message. The heart rate can be acquired through a known procedure based on the electrocardiographic waveform. The process proceeds to step S18 after the process of step S12 is terminated.

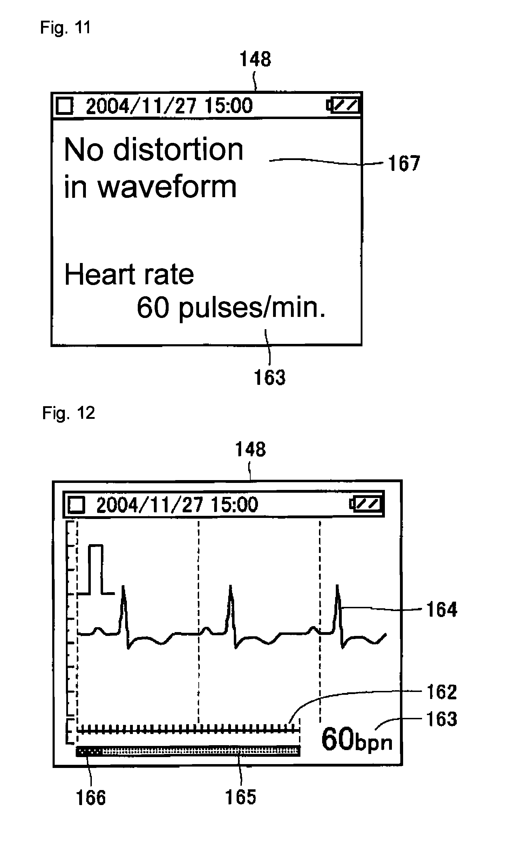

A screen display example in step S12 is shown in Fig. 11. In Fig. 11, a message 167 "no distortion in waveform" and data 163 of the heart rate per unit time of "60 pulses/min." are displayed on the display unit 148.

In step S14, the CPU 154 displays a notification that the waveform analysis is not possible, and also displays a message on whether or not to save the measurement data on the display unit 148. Then, whether or not the subject has selected to save the measurement data according to the message is determined (step S16). If selection is made to save the measurement data (YES in step S16), the process proceeds to step S18. If selection is made to discard the measurement data (NO in step S16), the process proceeds to step S20.

In step S18, the CPU 154 performs a process of saving the measurement data. That is, the CPU 154 stores the current date and time data input from the timer 170, the electrocardiographic waveform data temporarily stored in the RAM, and the analysis result in correspondence to each other in a predetermined storage region of the memory 155. On the other hand, in step S20, the CPU 154 discards the measurement data.

The series of processes is terminated after the process of step S18 or step S20 is terminated.

Here, the portable electrocardiograph 100 has a function of reading out the measurement/analysis result of the electrocardiographic waveform saved in step S18 and displaying the same on the display unit 148. Fig. 12 shows one example of a display screen of the measurement result of the electrocardiographic waveform stored in the memory 155.

With reference Fig. 12, a reduced waveform 162 of the entire waveform over the entire measurement period is displayed at the lower level of the screen at the display starting point, and an enlarged waveform 164 of for example, two seconds after the start of measurement is displayed at the upper level thereof. A scale bar 165 showing the length of the measurement period is displayed below the reduced waveform 162. A pointer 166 for instructing which waveform of which part of the measurement period the enlarged waveform 164 is related to is displayed on the scale bar 165. Change on which waveform of which part of the entire measurement period to display as the enlarged waveform 164 can be made in units of two seconds by operating the right scroll button 146 and the left scroll button 145, and moving the pointer 166 on the scale bar 165. When the subject pushes the determination button 144 after checking the electrocardiographic waveform on the screen of Fig. 12, the CPU 154 reads out the data of the analysis result corresponding to the electrocardiographic waveform being displayed from the memory 155, edits the same to a message, and displays the edited message on the display unit 148 as shown in Fig. 11.

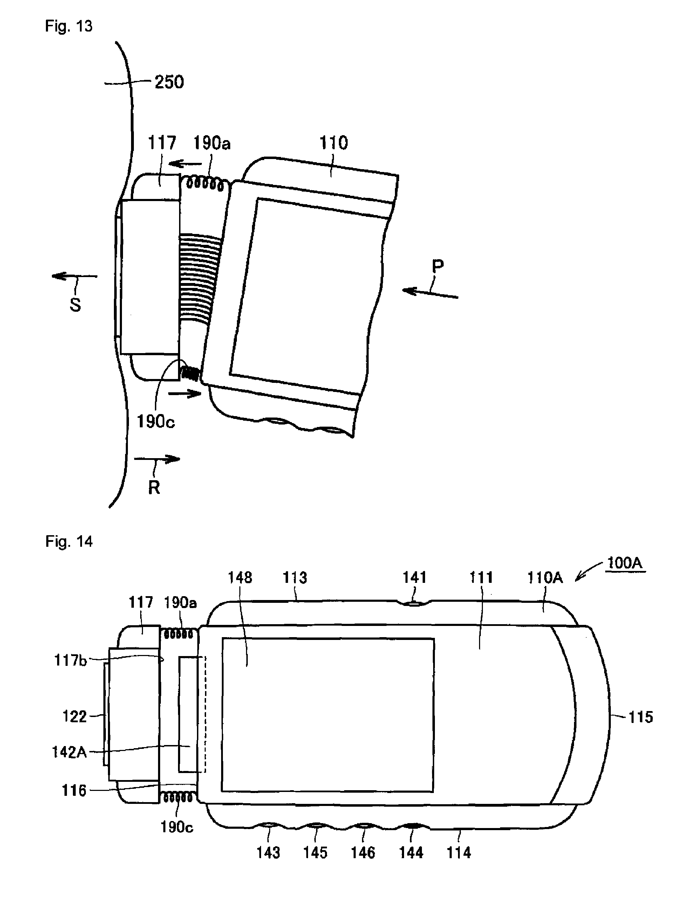

As described above, the springs 190a, 190b, 190c, and 190d for supporting the electrode unit 117 in a freely oscillating manner are arranged on the body unit 110 in the portable electrocardiograph 100 of the present embodiment. A state in which the springs 190a, 190b, 190c, and 190d are supporting the electrode unit 117 in a freely oscillating manner is shown in Fig. 13.

A coupling state of the body unit 110 and the electrode unit 117 of when the state of measuring the electrocardiographic waveform using the portable electrocardiograph 100 is seen from above is shown in Fig. 13. Suppose the moving direction (direction of arrow P in the figure) of the body unit 110 is shifted from the direction coinciding with the normal direction (direction of arrow R in the figure) at the surface of the chest region 250 or the measurement site. In this case, the electrode unit 117 oscillates as the spring 190c (and 190d) compresses and the spring 190a (and 190b) stretches, as shown in Fig. 13. Therefore, the contacting direction (direction of arrow S in the figure) of the positive electrode 122 arranged on the electrode unit 117 coincides with the normal direction at the surface of the chest region 250 irrespective of the moving direction of the body unit 110.

Even if the moving direction is the direction that coincides with the normal direction at the surface of the chest region 250, the direction in which the body unit 110 (left side face 116 thereof) is pressed towards the chest region 250 side (electrode unit 117 side) might shift if the hand or the arm gripping the body unit 110 lowers, for example, during the measurement. In such case as well, the shift in the direction of pressing the body unit 110 to the chest region 250 can be absorbed by the springs 190a, 190b, 190c, and 190d.

As described above, according to the portable electrocardiograph 100 of the present embodiment, the contacting direction of the positive electrode 122 can be coincided with the normal direction at the surface of the chest region 250 even if the moving direction or the pressing direction of the body unit 110 shifts from the normal direction at the surface of the chest region 250. That is, the electrode unit 117 can flexibly oscillate even if the left side face 116 of the body unit 110 and the surface of the measurement site are in a non-parallel state, whereby the contacting face 122a of the positive electrode 122 and the surface of the measurement site can be maintained parallel to each other.

The contact stability between the positive electrode 122 and the measurement site is thus enhanced, and variation in the measurement voltage value is prevented from occurring. Therefore, the electrocardiographic waveform can be accurately and stably measured according to the configuration described above. Note that, in Fig. 13, an example where the moving direction is shifted towards the lower face 114 side of the body unit 110 has been shown, but similar effects are obtained even if the moving direction is shifted towards the upper face 113 side, the front face 111 side, or the rear face 112 side of the body unit 110.

Four springs are arranged in the portable electrocardiograph 100 according to the present embodiment, as described above, but the number of springs is not limited to four as long as it is arranged between the body unit 110 and the electrode unit 117 and can support the electrode unit 117 in a freely oscillating manner. One spring for coupling a position at substantially the middle of the left side face 116 of the body unit 110 and a position at substantially the middle of the face 117b of the electrode unit 117 may be arranged.

In the portable electrocardiograph 100 according to the present embodiment, a case of using a coil spring as an example of an elastic body has been illustrated, but the elastic body is not limited to the coil spring, and may be rubber, or the like.

In the portable electrocardiograph 100 according to the present embodiment, a case in which the measurement site to contact the positive electrode 122 is the chest region 250 has been illustrated and described, but the measurement site to contact the positive electrode 122 is not limited to the chest region 250, and may be four limbs or the torso other than the hand that grips the portable electrocardiograph 100.

### [Second embodiment]

The subject needed to push the measurement button 142 to start the measurement in the portable electrocardiograph 100 of the first embodiment. In a portable electrocardiograph 100A according to the present embodiment, on the other hand, the measurement is automatically started using the configuration of the portable electrocardiograph of the present invention. The basic hardware configuration and operation of the portable electrocardiograph 100A according to the present embodiment are similar to those of the portable electrocardiograph 100 of the first embodiment described above. Therefore, description is made with the same reference numerals denoted for the same or corresponding portions.

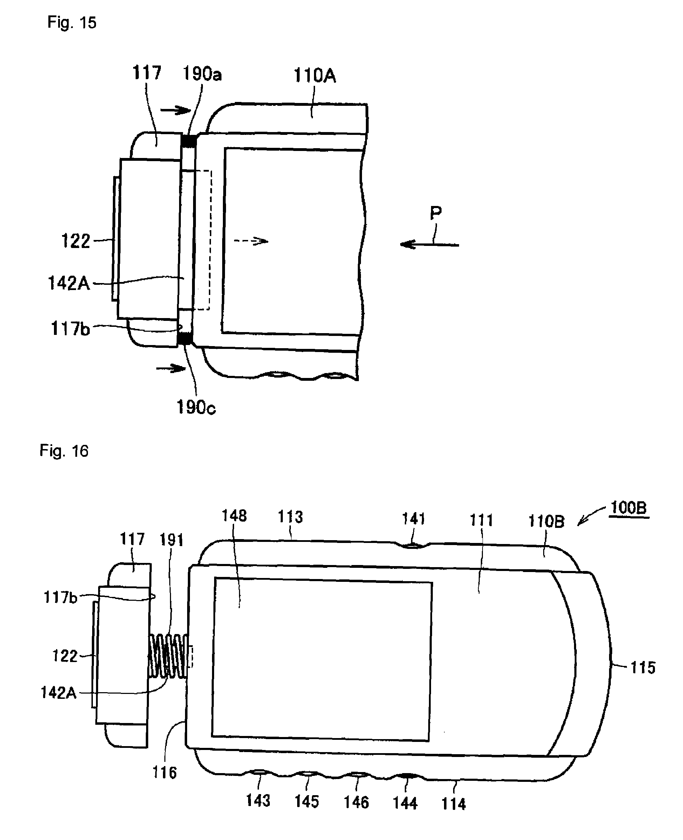

The portable electrocardiograph 100A according to the present embodiment will now be described focusing on the difference with the portable electrocardiograph 100 of the first embodiment using Figs. 14 and 15. Here, the illustration of the flexible substrate 180 is omitted in Figs. 14 and 15.

One example of an outer appearance of the portable electrocardiograph 100A according to the present embodiment is shown in Fig. 14. With reference to Fig. 14, in the portable electrocardiograph 100A according to the present embodiment, a measurement button 142A is arranged on the left side face 116 of the body unit 110A so as to project out towards the face 117b side of the electrode unit 117 in the unused state in place of the measurement button 142 of the first embodiment. A switch and the like (hereinafter referred to as "push detection switch) (not shown) for detecting whether or not the measurement button 142A is pushed is assumed to be arranged inside the body unit 110A. The push detection switch instructs start of measurement of the electrocardiographic waveform to the CPU 154 when determining that the measurement button 142A is pushed.

A state in which the measurement button 142A is pushed in the portable electrocardiograph 100A of the present embodiment is shown in Fig. 15. In the figure, a case where the moving direction (direction of arrow P in the figure) of the body unit 110A coincides with the normal direction at the surface of the chest region 250 is shown. With reference to Fig. 15, suppose the subject brings the body unit 110A closer to the measurement site (not shown) in the direction indicated by the arrow P in the figure in time of use (in time of measurement). The springs 190a, 190b, 190c, and 190d then compress. The measurement button 142A is accordingly pushed by the face 117b of the electrode unit 117 in the direction indicated by an arrow of broken line in the figure (direction opposite to the moving direction). The push detection switch (not shown) in the body unit 110A then detects that the measurement button 142A is pushed, and instructs the CPU 154 to start the measurement.

In the portable electrocardiograph 100A of the present embodiment, the CPU 154 determines whether or not a detection signal from the push detection switch (not shown) is input in step S5 of the flowchart of Fig. 10. If the detection signal is input, the CPU 154 assumes that the instruction to start the measurement is made (YES in step S5), and executes the processes of step 6 and thereafter of Fig. 10.

As described above, in the portable electrocardiograph 100A of the present embodiment, when the positive electrode 122 is pressed against the measurement site as a result of the body unit 110A being brought close to the measurement site, the distance between the body unit 110A and the electrode unit 117 becomes closer and the measurement button 142A is pushed. Even if the actual moving direction of the body unit 110A and the normal direction at the surface of the measurement site are shifted, at least one of the springs 190a, 190b, 190c, or 190d is desirably compressed so that the measurement button 142A is pushed. Thus, as shown in Figs. 14 and 15, the single measurement button 142A is preferably arranged over a wide range on the inner side of the positions arranged with the four springs 190a, 190b, 190c, and 190d at the left side face 116 of the body unit 110A. Four measurement buttons may be arranged in the axial direction of the respective springs 190a, 190b, 190c, and 190d at the positions arranged with the springs 190a, 190b, 190c, and 190d at the left side efface 116 of the body unit 110A.

### (Variant)

In the portable electrocardiograph 100A of the present embodiment, description has been made illustrating a configuration in which a plurality of springs is arranged between the body unit and the electrode unit, similar to the portable electrocardiograph 100 according to the first embodiment described above. However, the operation similar to the above is possible even if only one spring is arranged between the body unit and the electrode unit. Fig. 16 shows one example of an outer appearance of a portable electrocardiograph 100B according to a variant of the present embodiment. Other configurations are the same as the portable electrocardiograph 100A of the second embodiment described above.

As shown in Fig. 16, in the portable electrocardiograph 100B according to the present variant, the face 117b of the electrode unit 117 and the left side face 116 of the body unit 110B are coupled with one spring 191. More specifically, the spring 191 couples the position at substantially the middle of the face 117b and the position at substantially the middle of the left side face 116. The spring 191 supports the electrode unit 117 in a freely oscillating manner. The measurement button 142A is arranged in a hollow part of the interior portion of the spring 191. In this case as well, the measurement button 142A is arranged on the side face 116 of the body unit 110B so as to project out towards the face 117b side of the electrode unit 117 in the unused state, similar to the portable electrocardiograph 100A of the second embodiment described above.

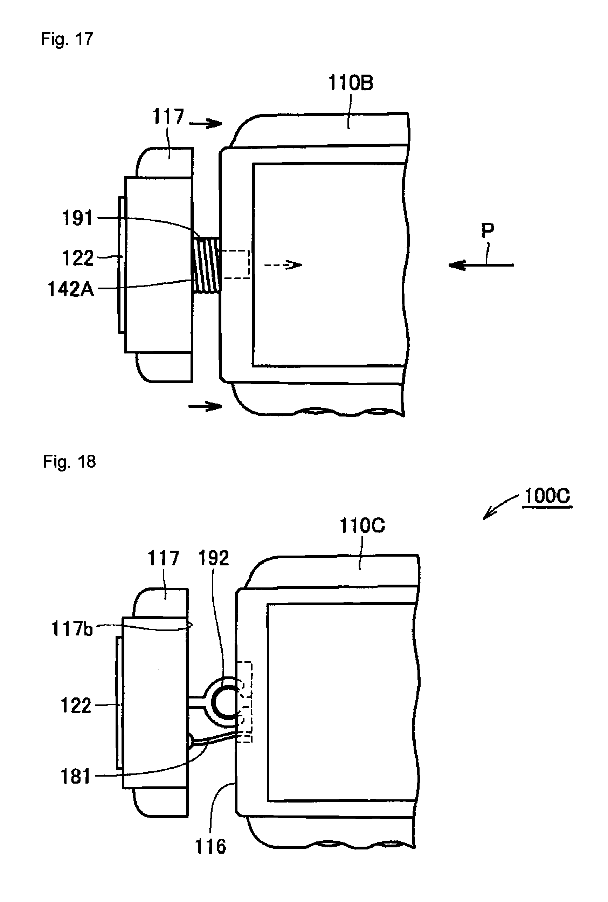

A state in which the measurement button 142A is pushed in the portable electrocardiograph 100B of the present variant is shown in Fig. 17. With reference to Fig. 17, suppose the subject moves the body unit 110B in the direction indicated by arrow P in the figure with respect to the measurement site (not shown) in time of use (in time of measurement). The spring 191 then compresses. The measurement button 142A is then pushed in the direction indicated by an arrow of broken line in the figure (direction opposite to the moving direction) by the face 117b of the electrode unit 117. The push detection switch (not shown) in the body unit 110B then detects that the measurement button 142A is pushed, and instructs start of measurement to the CPU 154. Thus, the measurement of the electrocardiographic waveform automatically starts in the portable electrocardiograph 100B according to the present variant.

Note that, in the portable electrocardiographs 100A, 100B of the second embodiment described above and the variant thereof, description has been made that the measurement starts when the measurement button 142A is pushed, but in addition to or in place thereof, information indicating the contacting state of the positive electrode 122 may be informed to the subject based on whether or not the measurement button 142A is pushed. For instance, a message "good contacting state" may be displayed on the display unit 148 or the information of the contacting state may be informed by voice.

### [Third embodiment]

A third embodiment of the present invention will now be described. A case where a stretchable elastic body (spring) is used for the supporting body has been described in the portable electrocardiographs 100, 100A, 100B of the first and the second embodiments. In a portable electrocardiograph 100C according to the present embodiment, a universal joint (universal bearing) is used for the supporting body. The basic hardware configuration and operation of the portable electrocardiograph 100C of the present embodiment are the same as the portable electrocardiograph 100 of the first embodiment described above. Therefore, same references are denoted for the same or corresponding portions.

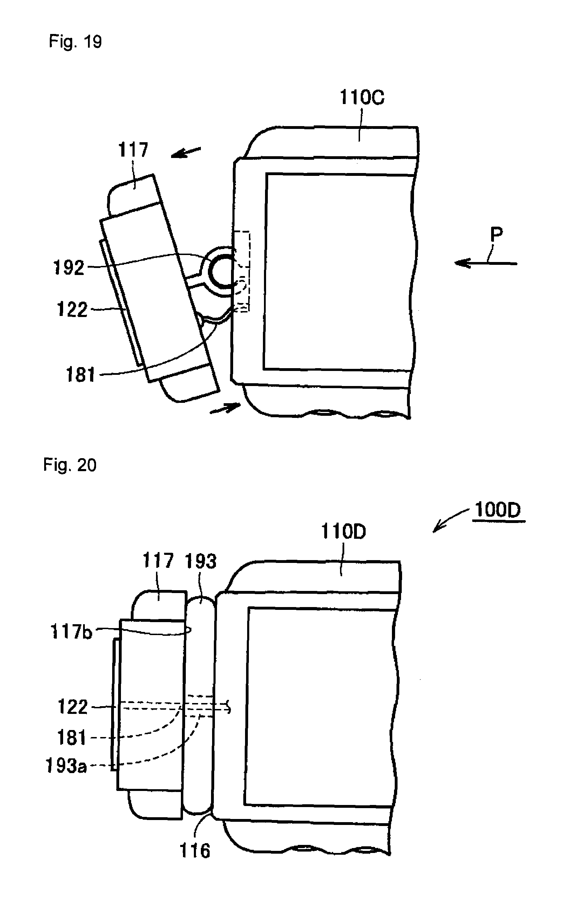

The portable electrocardiograph 100C of the present embodiment will now be described focusing on the difference with the portable electrocardiograph 100 of the first embodiment using Figs. 18 and 19.

Fig. 18 is a view showing one part of an outer appearance of the portable electrocardiograph 100C of the present embodiment. With reference to Fig. 18, in the portable electrocardiograph 100C of the present embodiment, the face 117b of the electrode unit 117 and the left side face 116 of the body unit 110C are coupled by a universal joint 192. More specifically, the universal joint 192 couples the position at substantially the middle of the face 117b and the position at substantially the middle of the left side face 116. Here, a concave part is formed at the position to be arranged with the universal joint 192 in the left side face 116 of the body unit 110C, as shown in Fig. 18, so that the portable electrocardiograph 100C does not become longer than necessary.

In the portable electrocardiograph 100C of the present embodiment, the positive electrode 122 arranged in the electrode unit 117 and the processing circuit 150 in the body unit 110C are electrically connected by a lead wire 181 and the like.

A state in which the universal joint 192 is supporting the electrode unit 117 in a freely oscillating manner in the portable electrocardiograph 100C of the present embodiment is shown in Fig. 19. In Fig. 19, the coupling state of the body unit 110C and the electrode unit 117 of when a state in which the electrocardiographic waveform is measured using the portable electrocardiograph 100C is seen from above is shown. The universal joint 192 tilts when the body unit 110C is moved or pushed in a direction (direction of arrow P in the figure) shifted from the normal direction at the surface of the measurement site with respect to the measurement site (not shown), as shown in Fig. 19. Therefore, even if the left side face 116 of the body unit 110C and the surface of the measurement site are in a non-parallel state, the positive electrode 122 arranged in the electrode unit 117 and the surface of the measurement site can be maintained parallel to each other.

Thus, effects similar to the portable electrocardiograph 100A of the first embodiment described above can be obtained in the portable electrocardiograph 100C of the present embodiment.

Similar to the portable electrocardiograph 100A of the second embodiment described above, a configuration of detecting whether or not the positive electrode 122 is pressed against the measurement site may be adopted even if the universal joint 192 is used for the supporting member as in the portable electrocardiograph 100C of the present embodiment. For instance, an acceleration sensor (not shown) for detecting change in position of the electrode unit 117 may be arranged in the electrode unit 117 to realize the above configuration. For instance, after the routine shown in Fig. 10 is started, the CPU 154 determines whether or not the detection amount (value) based on the detection signal from the acceleration sensor (not shown) is within a predetermined value in step S5. If the detection amount is within the predetermined value, oscillation of the electrode unit 117 is assumed to be stopped, and thus determination is made that the positive electrode 122 arranged in the electrode unit 117 is pressed against the measurement site.

### [Fourth embodiment]

A fourth embodiment of the present invention will now be described. A case where an elastic body (coil spring) is used for the supporting member has been described in the portable electrocardiographs 100, 100A, 100B of the first and the second embodiments. A case where a universal joint is used for the supporting member has been described in a portable electrocardiograph 100C according to the third embodiment. In a portable electrocardiograph 100D according to the present embodiment, a fluid bag is used for the supporting member. Here, the basic hardware configuration and operation of the portable electrocardiograph 100D of the present embodiment are the same as the portable electrocardiograph 100 of the first embodiment described above. Therefore, same references are denoted for the same or corresponding portions.

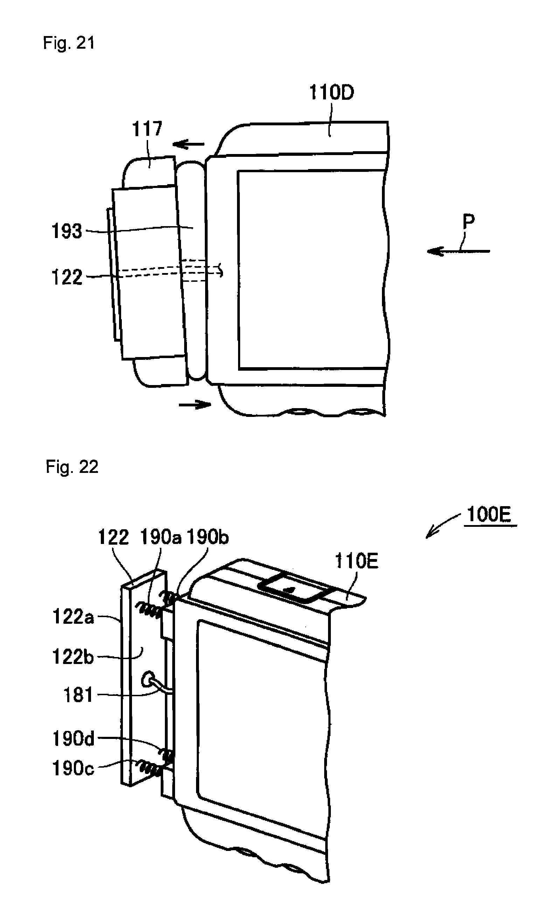

The portable electrocardiograph 100D of the present embodiment will now be described focusing on the difference with the portable electrocardiograph 100 of the first embodiment using Figs. 20 and 21.

Fig. 20 is a view showing one part of an outer appearance of the portable electrocardiograph of the fourth embodiment of the present invention. With reference to Fig. 20, in the portable electrocardiograph 100D of the present embodiment, the face 117b of the electrode unit 117 and the left side face 116 of the body unit 110D are coupled by a fluid bag 193. The fluid bag 193 is adhered to substantially the entire face of both the face 117b of the electrode unit 117 and the left side face 116 of the body unit 110D. In the portable electrocardiograph 100D according to the present embodiment, the fluid bag 193 is formed to an annular shape (doughnut shape) having a hole 193a at substantially the center, where a predetermined position of the face 117b of the electrode unit 117 and a predetermined position of the left side face 116 of the body unit 110D face each other through the hole 193a. The lead wire 181 for electrically connecting the positive electrode 122 arranged in the electrode unit 117 and the processing circuit 150 in the body unit 110D is arranged through the hole 193a. Note that, the hole 193a for connecting the lead wire 181 is formed in the fluid bag 193 in the above description, but it is not limited to such mode. For instance, the lead wire 181 may be arranged to lie along the surface of the fluid bag 193.

A state in which the fluid bag 193 is supporting the electrode unit 117 in a freely oscillating manner in the portable electrocardiograph 100D of the present embodiment is shown in Fig. 21. In Fig. 21, the coupling state of the body unit 110D and the electrode unit 117 of when a state in which the electrocardiographic waveform is measured using the portable electrocardiograph 100D is seen from above is shown. The fluid bag 193 deforms when the body unit 110D is moved or pushed in a direction (direction of arrow P in the figure) shifted from the normal direction at the surface of the measurement site with respect to the measurement site (not shown), as shown in Fig. 21. Therefore, even if the left side face 116 of the body unit 110D and the surface of the measurement site are in a non-parallel state, the positive electrode 122 arranged in the electrode unit 117 and the surface of the measurement site can be maintained parallel to each other.

Thus, effects similar to the portable electrocardiograph 100 of the first embodiment described above can be obtained in the portable electrocardiograph 100D of the present embodiment.

Note that, similar to the portable electrocardiograph 100A of the second embodiment described above, a configuration of detecting whether or not the positive electrode 122 is pressed against the measurement site may be adopted even if the fluid bag 193 is used for the supporting member as in the portable electrocardiograph 100D of the present embodiment. For instance, a pressure sensor (not shown) may be arranged in the fluid bag 193 to realize the above configuration. For instance, after the routine shown in Fig. 10 is started, the CPU 154 determines whether or not the detection amount (value) based on the detection signal from the pressure sensor (not shown) is within a predetermined value in step S5. If the detection amount is within the predetermined value, oscillation of the electrode unit 117 is assumed to be stopped, and thus determination is made that the positive electrode 122 arranged in the electrode unit 117 is pressed against the measurement site.

### [Fifth embodiment]

A fifth embodiment of the present invention will now be described. A case where the supporting member couples the body unit and the electrode unit has been described in the portable electrocardiographs 100, 100A to 100D of the first to the fourth embodiments. In a portable electrocardiograph 100E of the present embodiment, the supporting member directly couples the body unit and the positive electrode. The basic hardware configuration and operation of a portable electrocardiograph 100E of the present embodiment are the same as the portable electrocardiograph 100 of the first embodiment described above. Therefore, same references are denoted for the same or corresponding portions.

The portable electrocardiograph 100E of the present embodiment will now be described focusing on the difference with the portable electrocardiograph 100 of the first embodiment using Figs. 22 to 24.

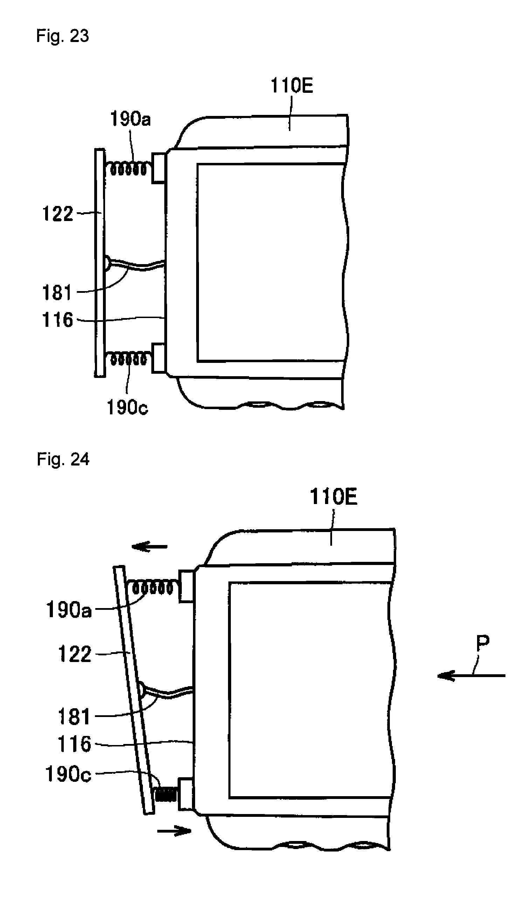

Fig. 22 is a perspective view showing one part of an outer appearance of the portable electrocardiograph 100E of the present embodiment. Fig. 23 is a front view showing one part of an outer appearance of the portable electrocardiograph 100E of the present embodiment.

With reference to Figs. 22 and 23, the left side face 116 of the body unit 110E and the face facing the body unit 110E of the positive electrode 122 (i.e., face positioned on the side opposite to the measurement site contacting face 122a) 122b are coupled by the springs 190a, 190b, 190c, and 190d in the portable electrocardiograph 100E of the present embodiment. More specifically, the face 122b of the positive electrode 122 has a substantially rectangular shape, and the springs 190a, 190b, 190c, and 190d couple the respective four corners of the face 122b of the positive electrode 122 and the left side face 116 of the body unit 110E. The lead wire 181 for electrically connecting the positive electrode 122 and the processing circuit 150 in the body unit 110E is connected between the face 122b of the positive electrode 122 and the left side face 116 of the body unit 110E.

A state in which the springs 190a, 190b, 190c, and 190d are supporting the positive electrode 122 in a freely oscillating manner in the portable electrocardiograph 100E of the present embodiment is shown in Fig. 24.

In Fig. 24, the coupling state of the body unit 110E and the positive electrode 122 of when a state in which the electrocardiographic waveform is measured using the portable electrocardiograph 100E is seen from above is shown. The spring 190c (and spring 190d) compresses, and the spring 190a (and 190b) stretches, for example, when the body unit 110E is moved or pushed in a direction (direction of arrow P in the figure) shifted from the normal direction at the surface of the measurement site with respect to the measurement site (not shown), as shown in Fig. 24. Therefore, even if the left side face 116 of the body unit 110E and the surface of the measurement site are in a non-parallel state, the positive electrode 122 (contacting face 122a thereof) and the measurement site can be maintained parallel to each other.

Thus, effects similar to the portable electrocardiograph 100 of the first embodiment described above can be obtained in the portable electrocardiograph 100E of the present embodiment.

Note that, in the portable electrocardiograph 100E according to the present embodiment, a case of arranging four springs has been described, but the number of springs is not limited to four and, for example, only one spring may be arranged or five or more springs may be arranged. Alternatively, in place of the spring, other elastic bodies, or universal joint or fluid bag may be arranged.

Similar to the portable electrocardiograph 100A of the second embodiment described above, the measurement button 142A may be arranged on the left side face 116 of the body unit 110E to automatically start the measurement of the electrocardiographic waveform in the portable electrocardiograph 100E of the present embodiment. Alternatively, the contacting state of the positive electrode 122 may be informed to the subject.

The embodiments disclosed herein are merely illustrative in all aspects and should not be construed as restrictive. The scope of the invention is defined by the appended claims rather than by the description preceding them, and all changes that fall within meets and bounds of the claims, or equivalence of such meets and bounds are therefore intended to be embraced by the claims.

## Claims

1. A portable electrocardiograph comprising:
a first electrode to be contacted by a hand of a subject;
a second electrode to be contacted to a predetermined measurement site of the subject;
a measurement unit for measuring an electric signal detected by the first electrode and the second electrode as an electrocardiographic waveform;
a first housing including the first electrode and the measurement unit; and
an oscillating and supporting mechanism, arranged in the first housing, for supporting the second electrode in a freely oscillating manner.

2. The portable electrocardiograph according to claim 1, wherein the first housing has an electrode formed face arranged with the first electrode, and a first face positioned on the opposite side of the electrode formed face;
the second electrode has a contacting face which contacts the measurement site, and a second face positioned on the opposite side of the contacting face; and
the oscillating and supporting mechanism couples the first face and the second face.

3. The portable electrocardiograph according to claim 2, further comprising a detection unit for detecting whether or not the second electrode is pressed against the measurement site.

4. The portable electrocardiograph according to claim 3, wherein the detection unit includes a button arranged on the first face and projected out towards the second face side in an unused state, and a push detection unit for detecting whether or not the button is pushed; and
the detection unit instructs start of measurement by the measurement unit when the pushing of the button is detected.

5. The portable electrocardiograph according to claim 1, further comprising:
a second housing arranged with the second electrode; and
the oscillating and supporting mechanism couples the first housing and the second housing.

6. The portable electrocardiograph according to claim 5, wherein the first housing has a first electrode formed face arranged with the first electrode, and a first face positioned on the opposite side of the first electrode formed face;
the second housing has a second electrode formed face arranged with the second electrode, and a second face positioned on the opposite side of the second electrode formed face; and
the oscillating and supporting mechanism couples the first face and the second face.

7. The portable electrocardiograph according to claim 6, further comprising a detection unit for detecting whether or not the second electrode is pressed against the measurement site.

8. The portable electrocardiograph according to claim 7, wherein the detection unit includes a button arranged on the first face and projected out towards the second face side in an unused state, and a push detection unit for detecting whether or not the button is pushed; and
the detection unit instructs start of measurement by the measurement unit when the pushing of the button is detected.

9. The portable electrocardiograph according to claim 1, wherein the oscillating and supporting mechanism includes an elastic body.

10. The portable electrocardiograph according to claim 1, wherein the oscillating and supporting mechanism includes a fluid bag containing fluid.

11. The portable electrocardiograph according to claim 1, wherein the oscillating and supporting mechanism includes a universal joint.
